# EUROPEAN PATENT APPLICATION

(11) **EP 2 561 859 A1**
(43) Date of publication of application: **27.02.2013**
(21) Application number: 11178771.9
(22) Date of filing: 25.08.2011
(51) Int. Cl.: A61K 8/81, A61Q 5/12, C08F 226/10, C08F 290/06

(54) **Rheology modifiers for surfactant formulations**

(71) Applicant: BASF SE, 67056 Ludwigshafen (DE)
(72) Inventor: Nguyen-Kim, Son, 69502 Hemsbach (DE); Werner, Rolf, 67069 Ludwigshafen (DE); Schmitt, Angelika, 67551 Worms (DE); Wood, Claudia, 69469 Weinheim (DE); Nguyen-Kim, Viet, 69502 Hemsbach (DE); Rausch, Monika, 67125 Darmstadt-Schauernheim (DE)
(74) Representative: Reinhardt, Jürgen

(57) **Abstract**

Use of a water-swellable copolymer consisting essentially of the following monomers in polymerized form
a) vinylpyrrolidone and/or methacrylamide (monomer A);
b) one or more monomers of the formula I

R¹-O-(CH₂-CHR-O)ₙ-R² (I)

wherein,
R¹ is a (meth)acrylic radical,
R is H or CH₃,
R² is a linear or branched C₈ to C₃₀ saturated hydrocarbon radical,
n is an integer of from 5 to 100
(monomer B);
c) vinylimidazole, quaternized vinylimidazole, N,N-dimethylaminopropyl-methacrylamide, quaternized N,N-dimethylaminoethyl (meth)acrylate, trimethylaminoethylmethacrylate chloride or a mixture of two or more thereof (monomer C);
d) (meth)acrylic acid and/or methyl(meth)acrylate (monomer D);
e) from 0 to 0.5% by weight of one or more cross-linkers (monomer E), based on the total weight of the monomers A to E,

as a rheology-modifier in a formulation comprising at least one surfactant and/or an oil.

## Description

The invention relates to the use of a water-swellable copolymer which essentially consists of the following monomers in polymerized form
a) vinylpyrrolidone and/or methacrylamide (monomer A);
b) one or more monomers of the formula I

   R¹-O-(CH₂-CHR-O)ₙ-R² (I)

   wherein,
   R¹ is a (meth)acrylic radical,
   R is H or CH₃,
   R² is a linear or branched C₈ to C₃₀ saturated hydrocarbon radical,
   n is an integer of from 5 to 100
   (monomer B);
c) vinylimidazole, quaternized vinylimidazole, N,N-dimethylaminopropyl-methacrylamide, quaternized N,N-dimethylaminoethyl (meth)acrylate, trimethylaminoethylmethacrylate chloride or a mixture of two or more thereof (monomer C);
d) (meth)acrylic acid and/or methyl(meth)acrylate (monomer D);
e) from 0 to 0.5% by weight of one or more cross-linkers (monomer E),
based on the total weight of the monomers A to E,
as a rheology-modifier in a formulation comprising at least one surfactant and/or an oil. The oil used is preferably a cosmetically acceptable oil.

The invention further relates to a process for the preparation of water-swellable copolymers wherein the monomers are polymerized by means of precipitation polymerization in cyclohexane and/or ethyl acetate.

The invention further relates to a cosmetic formulation which comprises
α) at least one said water-soluble copolymer,
β) at least one surfactant,
γ) at least one cosmetically acceptable active ingredient and/ or auxiliary different from α).

specification. Combinations of preferred embodiments are within the scope of the present Preferably the cosmetic formulation is an emulsion, in particular a cream or a lotion or a cleansing composition, in particular a shampoo or a body wash. Further preferred embodiments are disclosed in the claims and the invention.

Specific requirements are often placed on formulations which comprise surfactants, in particular on cleansing compositions, and even more in the area of cosmetics, with regard to their rheological properties. They can often only be converted to the desired application form by using additives, which enable a modulation of their rheological properties to the desired application form. Such additives are most often so-called "thickeners". Examples of customary thickeners used in cleansing formulations are salts such as sodium chloride or cross-linked anionic polymeric thickeners. The use of a salt does not allow for a fine adjustment of the rheological behaviour of the formulation to be thickened. Besides, the presence of a salt in high amounts in cosmetic products is often associated with irritation of the skin and the eyes. In addition, anionic polymeric thickeners may be incompatible with cationically-charged conditioning polymers present in many formulations containing surfactants such as shampoos and body washes and thus lead to a destabilization of the formulation.

Copolymers have also been used as thickeners for cleansing compositions.

EP 1 069 142 A1 writes on a polymeric thickener with surfactant properties comprising a surfactant monomer and at least one ethylenically unsaturated monomer. According to the disclosure, the thickening effect of this polymer is said to appear when the formulation containing this polymer is heated above the LCST-temperature of the polymer.

EP 0 121 230 A1 discloses a copolymer containing an ethylenically unsaturated monomer, an ester of the crotonic acid and an ester of (meth)acrylic acid. This polymer is used for the thickening of dispersions and according to what is disclosed in this application has to be neutralised with a base in order for the thickening effect to take place.

EP 0 003 235 A1 describes a polymer made of a C₃-C₅ carboxylic acid or sulfonic acid-based monomer and a surfactant-like monomer. According to this application, the polymer is used as thickener and dispersing agent of pigments and has to be neutralized in order to be dissolved in water.

EP 0 011 806 A1 writes on a liquid emulsion polymer used as a pH responsive thickener for aqueous compositions comprising a C₃-C₈ carboxylic acid monomer, C₂-C₁₂ ethylenically unsaturated nonionic monomer and a nonionic surfactant monomer.

EP 1 913 038 A1 describes an ampholytic copolymer suitable for modifying the rheological properties of aqueous active substances, e.g. for cosmetic compositions, pharmaceutical compositions and hygiene products. The ampholytic copolymer consists of at least one compound with a free-radically polymerizable, α,β-ethylenically unsaturated double bond and at least one anionogenic and/or anionic group per molecule (monomer a), at least one compound which is chosen from N-vinylimidazole compounds, N-[3-(dimethylamino)propyl]acrylamide, N-[3-(dimethylamino)propyl]methacrylamide and mixtures thereof, at least one free-radically polymerizable crosslinking compound which comprises at least two α, β-ethylenically unsaturated double bonds per molecule.

EP 1 912 620 relates to the use of anionically and cationically ampholytic copolymers as rheology modifiers for hair cosmetic compositions. The cationically ampholytic copolymers comprises at least one compound with a free-radically polymerizable, α,β-ethylenically unsaturated double bond and at least one cationogenic and/or cationic group per molecule, at least one free-radically polymerizable crosslinking compound which comprises at least two α, β-ethylenically unsaturated double bonds per molecule, if appropriate in the presence of at least one silicone compound comprising a polyether group and/or a free-radically polymerizable olefinically unsaturated double bond.

It was the object of the present invention to find a rheology-modifier which would enable the thickening of a formulation containing surfactants and/or an oil whilst obtaining a stable formulation. A further object was to find a rheology-modifier which would allow to maintain an acceptable flow behaviour of the thickened liquid or gel thus obtained. Another object was to synthesize a polymer readily soluble in an aqueous formulation and leading to a stable formulation whilst achieving the same thickening performance as with a conventional cross-linked polymeric thickener.

The problem was solved by using a water-swellable copolymer as defined above as a rheology-modifier in surfactant-containing formulations, in particular in cosmetic formulations comprising at least one surfactant.

Surprisingly, the copolymer according to the invention also enabled the preparation of mild cleansing formulations involving the use of mild surfactants or with a reduced amount of total surfactant systems, of formulations containing emollient oils, silicone or other lipophilic components as well as cationic conditioning polymers or pigments. Next to their thickening performance, the copolymers according to the invention also have a conditioning effect, thus leading to a conditioning of the hair and/or the skin when incorporated in cosmetic formulations. This conditioning effect is believed to be due to the amphoteric nature of the copolymer. It was also noticed that, when the copolymer according to the invention is present in the formulation, more care ingredients such as emollient oils, silicone or other lipophilic components could be incorporated in the formulation without jeopardising the stability of the formulation.

For the purpose of this invention the prefix (meth) written before a compound means the respective unsubstituted compound and/or the compound substituted by the methyl group. For instance, "(meth)acrylic acid" means acrylic acid and/or methacrylic acid, (meth)acrylate means acrylate and/or methacrylate, (meth)acrylamide means acrylamide and/or methacrylamide. The copolymer used according to the invention is a statistical copolymer. Its molecular weight can be either distributed broadly or narrowly. The copolymer according to this invention can also be a mixture of two or more different copolymers. These may differ in structure and/or in molecular weight distribution.

The copolymer which is used according to the invention is water-swellable. These copolymers are able to absorb a multiple of their weight of aqueous liquids and to retain this volume of liquid even upon exposure to external pressure.

For the purpose of the present invention, "modification of rheological properties" or "copolymer as a rheology-modifier" is understood in the broad sense. Thus, the copolymer used according to the invention is generally suitable for thickening the consistency of formulations, in particular of cosmetic formulations within a wide range. Depending on the basic consistency of the formulations, flow properties from low viscosity to solid (no longer flowable) may generally be achieved depending on the amount of copolymer used. "Modification of rheological properties" is therefore understood as meaning, inter alia, the increase in the viscosity of liquids, the improvement of the thixotropic properties of gels, the solidification of gels and waxes. The viscosity of the formulations containing surfactants is preferably in a range from 40mPa.s to about 60 000mPas. In one embodiment, the viscosity of the formulations will preferably lie in a range from 10 000mPa.s to 60 000mPa.s, particularly preferably from 15 000 to 40 000mPas. In another embodiment, the viscosity of the formulations will preferably lie in a range from 60mPa.s to 2000mPa.s.

### Monomer A

### Monomer A is N-vinylpyrrolidone and/or methacrylamide.

Monomer A is hydrophilic. Usually, monomer A has a solubility in water of at least 60 g/l at 20 °C, preferably of at least 80 g/l and in particular at least 100 g/l. For example monomer A may be dissolved in water at 20°C in an amount of up to 200 g/L or more.

### Monomer B

In general, monomer B is hydrophobic. Depending on the special combinations of the groups R, R¹, R² and n, the hydrophobicity of monomer B can be made more or less pronounced. Thus monomer B may be insoluble in water at 20°C, that is to say at the detection level.

Possible solvents for monomer B include, for example, (meth)acrylic esters, (meth)acrylic acids, alcohols, customary organic solvents, water and any desired mixture thereof. The solvents used are preferably (meth)acrylic acid or methylmethacrylate, more preferably methacrylic acid.

Suitable examples of monomer B are C₈-₃₀ alkyl (meth)acrylate, mono C₈-₃₀ alkyl terminated poly(ethylene glycol) (meth)acrylate, vinyl ester of aliphatic C₈₋₃₀ carboxylic acids, and vinyl C₁-₄ alkyl ether. Preferred monomers B are C₁-₃₀ alkyl (meth)acrylate and poly(ethylene glycol) (meth)acrylate.

Preferred C₁-₃₀ alkyl (meth)acrylates are linear or branched C₁-C₃₀-alkyl (meth)acrylates, such as methyl acrylate, methyl methacrylate, ethyl acrylate, n-butyl acrylate, n-hexyl acrylate, n-octyl acrylate, n-decyl acrylate, 2-ethylhexyl acrylat, 2-propylheptyl acrylate, lauryl acrylate, stearyl acrylate, n-hexyl methacrylate, n-octyl methacrylate, n-decyl methacrylate, 2-ethylhexyl methacrylate, 2-propylheptyl methacrylate, lauryl methacrylate and stearyl methacrylat. More preferred are C₄-₁₈ alkyl (meth)acrylates.

Preferred mono C₁-₂₂ alkyl terminated poly(ethylene glycol) (meth)acrylate contains 1 to 100, more preferably 5 to 50, and in particular 10 to 40 ethylene glycol moieties. More preferred is mono C₁₂-₃₀ alkyl terminated poly(ethylene glycol) (meth)acrylate, in particular mono C₁₆-₂₀ alkyl terminated poly(ethylene glycol) (meth)acrylate.

Examples of vinyl esters of aliphatic (linear or branched) C₁-₂₂ carboxylic acid are vinyl acetate, vinyl propionate, vinyl laurate, vinyl stearate, vinyl ester of neodecanoic acid (VeoVa^{®}-10) or vinyl ester of branched C₆₁₇ alkyl acid (VeoVa^{®}-9). Preferred vinyl esters of aliphatic C₁-₂₂ carboxylic acid are vinyl esters of aliphatic C₁₂-₂₂ carboxylic acid, such as vinyl laurate and vinyl stearate.

Examples of vinyl C₁-₄ alkyl ether are vinyl methyl ether or vinyl ethyl ether.

### Monomer C

Monomers C are vinylimidazole, quaternized vinylimidazole (QVI), N,N-dimethylaminopropylmethacrylamide (DMAPMAM), quaternized N,N-dimethylaminoethyl (meth)acrylate (Q-DMAEMA), trimethylaminoethylmethacrylate chloride (TMAEMC) or a mixture of two or more thereof, in case a mixture of two or three or more thereof is preferred. Preferably, comonomer C is vinylimidazol ("VI") and/or quaternized vinylimidazole.

The imidazoyl moieties of vinylimidazole can be quaternized. The conversion of monomer C to quaternary compounds can take place after the reaction. In the case of a subsequent conversion, the intermediate polymer can be isolated and purified first or converted directly. The conversion can be total or partial. Preferably at least 40mol%, particularly preferably at least 55mol% and very particularly preferably at least 70mol% of the incorporated monomers C are converted to the corresponding quaternary form. Usually up to 80mol%, particularly preferably up to 75mol% and very particularly preferably up to 60mol% of the incorporated monomers C are converted to the corresponding quaternary form.

Examples of suitable reagents for quaternizing monomer C are alkyl halides having 1 to 24 C atoms in the alkyl group, e.g. methyl chloride, methyl bromide, methyl iodide, ethyl chloride, ethyl bromide, propyl chloride, hexyl chloride, dodecyl chloride, lauryl chloride, propyl bromide, hexyl bromide, octyl bromide, decyl bromide, dodecyl bromide, methoxyethyl chloride and benzyl halides, especially benzyl chloride and benzyl bromide. Quaternization with long-chain alkyl radicals is preferably carried out with the corresponding alkyl bromides such as hexyl bromide, octylbromide, decylbromide, dodecyl bromide or lauryl bromide. Other suitable quaternizing agents are dialkyl sulfates, especially dimethyl sulfate or diethyl sulfate. The quaternization of the basic monomer C can also be carried out with alkylene oxides such as ethylene oxide or propylene oxide, in the presence of acids. Preferred quaternizing agents are methyl chloride or methoxyethyl chloride, methyl chloride being particularly preferred.

The quaternization of the monomer C or of the resulting copolymer with one of said quaternizing agents can be effected by generally known methods, or by methods available to a person skilled in the art.

Preferably, monomer C is used for the polymerization in predominantly cationogenic form, i.e. more than 80mol%, preferably more than 90mol%, particularly preferably more than 95mol% and very particularly preferably more than 99 mol% cationogenic, i.e. not in quaternized or protonated form, and only converted to the cationic or protonated form by quaternization during or, particularly preferably, after the polymerization.

In the case where the monomers are used in quaternized form, they can be used either as the dried substance, or in the form of concentrated solutions in solvents suitable for the monomers, e.g. in polar solvents such as water, methanol, ethanol or acetone, or in the other monomers C, provided these are suitable as solvents.

The resulting copolymers may be protonated. Protonation is understood as meaning that at least some of the protonatable groups of the polymer, preferably at least 20mol%, preferably more than 50mol%, particularly preferably more than 70mol% and very particularly preferably more than 90 mol%, are protonated, resulting in an overall cationic charge on the polymer. Water-soluble acids are particularly suitable for the protonation. Examples of compounds suitable for the protonation are mineral acids such as HCl and H₂SO₄ monocarboxylic acids, e.g. formic acid, lactic acid and acetic acid, dicarboxylic acids and polyfunctional carboxylic acids, e.g. oxalic acid and citric acid, and any other proton-donating compounds and substances that are capable of protonating the appropriate nitrogen atom.

### Monomer D

Monomer D can be acrylic acid, methacrylic acid, methylacrylate and/or methyl(meth)acrylate.

### Monomer E

The copolymers may comprise at least one crosslinker, i.e. a compound with two or more than two ethylenically unsaturated, non-conjugated double bonds in copolymerized form.

Preferably, monomer E is used in an amount of from 0 to 0.5% by weight, particularly preferably 0 to 0.2% by weight, based on the total weight of the monomers used for the polymerization. According to one embodiment, the copolymer is not crosslinked.

According to another embodiment, it is crosslinked. In the case where monomer E is used, it is typically employed in an amount of at least 0.01 wt% related to the total monomer amount.

As copolymer a mixture of crosslinked and not crosslinked copolymers may be employed as a rheology-modifier for further fine-tuning of the rheology.

Suitable monomers E are, for example, acrylic esters, methacrylic esters, allyl ethers or vinyl ethers of at least dihydric alcohols. The OH groups of the parent alcohols here may be completely or partially etherified or esterified; however, the crosslinkers comprise at least two ethylenically unsaturated groups.

Examples of the parent alcohols are dihydric alcohols, such as 1,2-ethanediol, 1,2-propanediol, 1,3-propanediol, 1,2-butanediol, 1,3-butanediol, 2,3-butanediol, 1,4-butanediol, but-2-ene-1,4-diol, 1,2-pentanediol, 1,5-pentanediol, 1,2-hexanediol, 1,6-hexanediol, 1,10-decanediol, 1,2-dodecanediol, 1,12-dodecanediol, neopentyl glycol, 3-methylpentane-1,5-diol, 2,5-dimethyl-1,3-hexanediol, 2,2,4-trimethyl-1,3-pentanediol, 1,2-cyclohexanediol, 1,4-cyclohexanediol, 1,4-bis(hydroxymethyl)cyclohexane, hydroxypivalic neopentyl glycol monoester, 2,2-bis(4-hydroxyphenyl)propane, 2,2-bis[4-(2-hydroxypropyl)phenyl]propane, diethylene glycol, triethylene glycol, tetraethylene glycol, dipropylene glycol, tripropylene glycol, tetrapropylene glycol, 3-thiopentane-1,5-diol, and polyethylene glycols, polypropylene glycols and polytetrahydrofurans with molecular weights of in each case 200 to 10 000. Apart from the homopolymers of ethylene oxide and propylene oxide it is also possible to use block copolymers of ethylene oxide or propylene oxide or copolymers which comprise incorporated ethylene oxide and propylene oxide groups. Examples of parent alcohols with more than two OH groups are trimethylolpropane, glycerol, pentaerythritol, 1,2,5-pentanetriol, 1,2,6-hexanetriol, triethoxycyanuric acid, sorbitan, sugars, such as sucrose, glucose, mannose. The polyhydric alcohols can of course also be used following reaction with ethylene oxide or propylene oxide as the corresponding ethoxylates or propoxylates. The polyhydric alcohols can also firstly be converted into the corresponding glycidyl ethers by reaction with epichlorohydrin. Preference is given to ethylene glycol di(meth)acrylate and polyethylene glycol di(meth)acrylates.

Further suitable monomers E are the vinyl esters or the esters of monohydric or polyhydric unsaturated alcohols with ethylenically unsaturated C₃-C₆-carboxylic acids, for example acrylic acid, methacrylic acid, itaconic acid, maleic acid or fumaric acid. Examples of such alcohols are allyl alcohol, 1-buten-3-ol, 5-hexen-1-ol, 1-octen-3-ol, 9-decen-1-ol, dicyclopentenyl alcohol, 10-undecen-1-ol, cinnamyl alcohol, citronellol, crotyl alcohol or cis-9-octadecen-1-ol. However, it is also possible to esterify the mono-hydric, unsaturated alcohols with polybasic carboxylic acids, for example malonic acid, tartaric acid, trimellitic acid, phthalic acid, terephthalic acid, citric acid or succinic acid.

Further suitable monomers E are esters of unsaturated carboxylic acids with the above-described polyhydric alcohols, for example of oleic acid, crotonic acid, cinnamic acid or 10-undecenoic acid.

Also suitable as monomers E are the acrylamides, methacrylamides and N-allylamines of at least difunctional amines. Such amines are, for example, 1,1-diaminomethane, 1,2-diaminoethane, 1,3-diaminopropane, 1,4-diaminobutane, 1,6-diaminohexane, 1,12-dodecanediamine, piperazine, diethylenetriamine or isophoronediamine. Likewise suitable are the amides of allylamine and unsaturated carboxylic acids, such as acrylic acid, methacrylic acid, itaconic acid, maleic acid, or at least dibasic carboxylic acids as have been described above.

It is of course also possible to use mixtures of the abovementioned monomers E.

As monomer E, very particular preference is given to ethylene glycol di(meth)acrylate, polyethylene glycol di(meth)acrylates, pentaerythritol triallyl ether, methylenebisacrylamide, N,N'-divinylethyleneurea, triallylamine and triallylmonoalkylammonium salts.

### Polymerisation

The copolymer to be used according to the invention can be prepared by conventional polymerization processes, e.g. by solution polymerization. The copolymer with particularly advantageous properties, i.e. with generally higher molecular weights and a better ability to form pulverulent formulations than are obtained by conventional polymerization processes is obtained by the preparation in accordance with the method of precipitation polymerization. The copolymer thus obtained is particularly advantageously suitable as rheology modifier (specifically thickener). A preferred embodiment of the invention is therefore the use of the copolymer which is obtainable by free-radical copolymerization by the method of precipitation polymerization in at least one organic solvent. In particular the precipitation polymerization according to the invention can be carried out in cyclohexane and/or ethyl acetate.

In the precipitation polymerization, the monomers used are soluble in the reaction medium whereas the corresponding copolymer is not. The copolymer which forms becomes insoluble under the polymerization conditions chosen and precipitates out of the reaction mixture.

The precipitation polymerization is preferably carried out in a largely anhydrous, aprotic solvent or solvent mixture, preferably in ethyl acetate and/or n-butyl acetate. A largely anhydrous, aprotic solvent or solvent mixture is understood as meaning a solvent or solvent mixture with a water content of at most 5% by weight.

The precipitation polymerization preferably takes place at a temperature in the range from 60 to 140°C, preferably from 65 to 100°C, in particular from 70 to 95°C. The resulting copolymer particles precipitate out of the reaction solution and can be isolated by customary methods, such as filtration using subatmospheric pressure.

The polymerization is usually carried out under atmospheric pressure, although it can also proceed under reduced or elevated pressure. A suitable pressure range is between 1 and 5 bar. Initiators

To prepare the copolymer which is used according to the invention, the monomers can be polymerized with the help of initiators which form free radicals.

Initiators for the free-radical polymerization which can be used are the peroxo and/or azo compounds customary for this purpose, for example alkali metal or ammonium peroxydisulfates, diacetyl peroxide, dibenzoyl peroxide, succinyl peroxide, di-tert-butyl peroxide, tert-butyl perbenzoate, tert-butyl perpivalate, tert-butyl peroxy-2-ethylhexanoate, tert-butyl permaleate, cumene hydroperoxide, diisopropyl peroxydicarbamate, bis(o-toloyl) peroxide, didecanoyl peroxide, dioctanoyl peroxide, dilauroyl peroxide, tert-butyl perisobutyrate, tert-butyl peracetate, di-tert-amyl peroxide, tert-butyl hydroperoxide, 2,2'-azobisisobutyronitrile, azobis(2-amidinopropane) dihydrochloride or 2-2'-azobis(2-methylbutyronitrile). Also suitable are initiator mixtures or redox initiator systems, such as, for example, ascorbic acid/iron(II) sulfate/sodium peroxodisulfate, tert-butyl hydroperoxide/sodium disulfite, tert-butyl hydroperoxide/sodium hydroxymethanesulfinate, H₂O₂/Cu^{I}.

### Cosmetic formulations

The water-soluble copolymer used according to the invention is specifically suitable as rheology modifiers for surfactant-containing formulations.

The copolymers are suitable both for the preparation of homogeneous-phase aqueous formulations, and also for the formulation of heterogeneous-phase compositions which additionally comprise at least one water-insoluble (hydrophobic) liquid or solid compound. "Homogeneous-phase compositions" have only a single phase irrespective of the number of their constituents. "Heterogeneous-phase compositions" are disperse systems of two or more immiscible components. These include solid/liquid, liquid/liquid and solid/liquid/liquid compositions, such as dispersions and emulsions, e.g. O/W and W/O formulations which have at least one of the oil or fat components described in more detail below and water as immiscible phases.

The invention further provides a cosmetic formulation comprising
α) at least one water-soluble copolymer as defined in any one of claims 1 to 10;
β) at least one surfactant;
γ) optionally, one or more cosmetically acceptable active ingredient and/ or auxiliary different from α).

In a preferred embodiment, the cosmetic formulation is a cosmetic cleansing formulation, i.e. a shampoo or a body wash.

In general for the cosmetic formulation according to the invention, the pH is adapted to its use in conjunction with the body. Preferably, the pH of the cosmetic formulation according to the invention is at least 3, more preferably at least 4. For example the pH may be at least 5, more preferably at least 5.5. Preferably, the pH is no more than 10, more preferably no more than 9 and most preferably no more than 8. The pH of the cosmetic formulation may also be adapted to that of the body and be no more than 7.

The cosmetic cleansing formulation of the present invention comprises the polymer component α) in an amount which suffices to increase the viscosity of the composition, preferably in an amount of from about 0.1 % to 8% by weight, based on the total weight of the formulation. Preferably, the amount of polymer in the cosmetic formulation is at least 0.5wt%, more preferably at least 0.75wt%, more preferably at least 1wt% and most preferably at least 1.2wt%.

The cosmetic formulation of the present invention preferably contains at least one surfactant, preferably from 1 % to 25% by weight of the total weight of the formulation. More preferably, the cosmetic cleansing formulation of the present invention contains at least 5wt% of at least one surfactant. Preferably, the cosmetic cleansing formulation of the present invention contains no more than 20wt% of at least one surfactant, more preferably no more than 15wt%.

The one or more surfactants, i.e. surfactant(s) β), are preferably selected from the groups of anionic surfactants such as alkyl sulfates, alkyl ether sulfates, alkylsulfonates, alkylarylsulfonates, alkyl succinates, alkyl sulfosuccinates, N-alkoyl sarcosinates, acyl taurates, acyl isethionates, alkyl phosphates, alkyl ether phosphates, alkyl ether carboxylates, alpha-olefinsulfonates, in particular the alkali metal and alkaline earth metal salts, e.g. sodium, potassium, magnesium, calcium, and ammonium and triethanolamine salts. The alkyl ether sulfates, alkyl ether phosphates and alkyl ether carboxylates can have between 1 and 10 ethylene oxide or propylene oxide units, preferably 1 to 3 ethylene oxide units, in the molecule.

These include, for example, sodium lauryl sulfate, ammonium lauryl sulfate, sodium lauryl ether sulfate, ammonium lauryl ether sulfate, sodium lauryl sarcosinate, sodium oleyl succinate, ammonium lauryl sulfosuccinate, sodium dodecylbenzenesulfonate, triethanolamine dodecylbenzenesulfonate.

Suitable amphoteric surfactant(s) are, for example, alkylbetaines, alkylamidopropylbetaines, alkylsulfobetaines, alkyl glycinates, alkyl carboxyglycinates, alkyl amphoacetates or -propionates, alkyl amphodiacetates or -dipropionates.

For example, cocodimethylsulfopropylbetaine, laurylbetaine, cocamidopropylbetaine or sodium cocamphopropionate can be used.

Suitable nonionic surfactant(s) are, for example, the reaction products of aliphatic alcohols or alkylphenols having 6 to 20 carbon atoms in the alkyl chain, which may be linear or branched, with ethylene oxide and/or propylene oxide. The amount of alkylene oxide is about 6 to 60 moles per mole of alcohol. In addition, alkylamine oxides, mono- or dialkylalkanolamides, fatty acid esters of polyethylene glycols, ethoxylated fatty acid amides, alkyl polyglycosides or sorbitan ether esters are suitable.

Furthermore, the shampoos can comprise cationic surfactants, such as quaternary ammonium compounds, for example cetyltrimethylammonium chloride.

In addition, the shampoo formulation according to the invention can comprise one or more thickeners, such as, for example, sodium chloride, PEG-55, propylene glycol oleate, PEG-120 methylglucose dioleate and others, and also one or more preservatives, further active ingredients and auxiliaries and water.

In the shampoo formulation according to the invention, in order to achieve certain effects, optionally one or more further ingredients γ) such as conditioners can be used. These conditioners include, for example, the cationic polymers with the INCI name Polyquaternium, in particular copolymers of vinylpyrrolidone/N-vinylimidazolium salts (Luviquat^{®} FC, Luviquat^{®} HM, Luviquat^{®} MS, Luviquat^{®} Care), copolymers of N-vinylpyrrolidone/dimethylaminoethyl methacrylate, quaternized with diethyl sulfate (Luviquat^{®} PQ 11), copolymers of N-vinylcaprolactam/N-vinylpyrrolidone/N-vinylimidazolium salts (Luviquat^{®} Hold); cationic cellulose derivatives (Polyquaternium-4 and -10), acrylamide copolymers (Polyquaternium-7). It is also possible to use protein hydrolysates, and conditioning substances based on silicone compounds, for example polyalkylsiloxanes, polyarylsiloxanes, polyarylalkylsiloxanes, polyethersiloxanes or silicone resins. Further suitable silicone compounds are dimethicone copolyols (CTFA) and aminofunctional silicone compounds, such as amodimethicone (CTFA). In addition, cationic guar derivatives such as guar hydroxypropyltrimonium chloride (INCI) can be used.

Suitable components γ) can also be nonionic polymers, siloxane-containing, water-soluble or water-dispersible polymers, e.g. polyether siloxanes, such as Tegopren^{®} (Goldschmidt) or Belsil^{®} (Wacker).

Further suitable components γ) are, for example, linear polydimethylsiloxanes, poly(methylphenylsiloxanes), cyclic siloxanes and mixtures thereof. The number-average molecular weight of the polydimethylsiloxanes and poly(methylphenylsiloxanes) is preferably in a range from about 1000 to 150 000 g/mol. Preferred cyclic siloxanes have 4- to 8-membered rings. Suitable cyclic siloxanes are commercially available, for example, under the name cyclomethicone.

A particular cosmetic cleansing composition in which the copolymer according to the invention is used is a body wash. Typical components of a body wash, in addition to the copolymer and surfactant mentioned previously, include sufficient base or acid to attain a pH of 4 to 6.75, preferably 4.5 to 6.75, and more preferably 5 to 6.5; and optional ingredients, including silicones, pearlizing agents, vitamins, oils, fragrances, dyes, biocides.

A second particular aqueous composition in which the polymer of this invention is useful is a shampoo. Typical components of a conditioning shampoo, in addition to the copolymer and surfactant mentioned previously, include sufficient base to attain a pH of 4.0 to 7.0, preferably, 5.0 to 7.0.

The copolymer used according to the invention is preferably suitable as a rheology-modifier for cleansing formulations. The copolymer enables the thickening of conventional cleansing formulations but also of cleansing formulations which comprise smaller amounts of surfactants: the total amount of surfactant in the formulation can be reduced by 30% up to 50%. than the conventional shampoos and body washes. They are thus advantageously suitable for the preparation of mild shampoos and baby shampoos.

The invention further provides a method for manufacturing optically clear aqueous formulations. The copolymer used according to the invention enables also a finer adjustment of the rheological properties of the cosmetic formulations.

The copolymer also leads to a conditioning effect of the hair and/or skin when incorporated in cosmetic formulations.

### Examples:

The invention is illustrated in more detail by reference to the following non limiting examples shown in table 1.

Abbreviations used:
- VP: Vinylpyrrolidon
- VI: Vinylimidazol
- LUMA: C₁₈-Alkyl-(EO)₂₅ -Methacrylat
- MAS: Methacrylsäure
- MA: Methylacrylat
- MMA: Methylmethacrylat
- CMP: 1-Chlor-3-methoxypropan
- LES: Sodium Laurylethersulfate
- CAPB: Cocoamidopropylbetaine

All polymers displayed in table 1 were prepared according to the following procedure:

### I) Polymer synthesis

### Preparation procedure for all polymers of Table 1

Synthesis example for polymer N° 4 (table 1) => VP/ VI / LUMA/ MAS /PETAE [56.8/36/3.6/ 3.6/ 0.15] (wt%)

| | | |
|---|---|---|
| **Initial charge** | 2100 g | Ethylacetate |
| | 8.5 g | Trigonox 101 |
| | | |
| Feed 1 | 280 g | Ethylacetate |
| | 481 g | Vinylpyrrolidone VP (monomer A) |
| | 306 g | Vinylimidazole VI (monomer C) |
| | 61,2 g | of a mixture consisting of 50% LUMA (monomer B)+ 45% MAS (monomer D1) + 5% MMA (monomer D2) |
| | 1.62 g | Pentaerythrittriallylether PETAE (monomer E) |
| | | |
| Feed 2 | 140 g | Ethylacetate |
| | 0.5g | tert.-Butylperoctoat |
| | | |
| Feed 3 | 330 g | Ethylacetate |
| | 2.5 g | tert.-Butylperoctoat |
| | 4.6 g | Trigonox 101 |
| | | |
| Feed 4 | 195 g | Methylchlorid |

The initial charge was heated to 85-88°C under a nitrogen atmosphere. Feed 1 and 2 were added over the course of 2 h. The mixture was stirred for a further 2 h at 88°C. Feed 3 was added in 30min and the mixture was then stirred in order to perform the post-polymerization for a further 3 h at 90°C. The resulting white suspension was quaternized with Methylchloride (Feed 4) in 1 h at 90°C. The obtained powder was filtered, washed twice with acetone and dried under reduced pressure at 40°C.

All of the products of table 1 were prepared analogously.

Polymer 6 was quaternized by using 1-Chlor-3-methoxypropan (CMP) at 80° for/ 5 h instead of Methylchloride.

### II) Tests in cosmetic cleansing formulations

A system composed of 10% LES and 4% CAPB, i.e. at a total surfactant concentration of 14wt%, was measured to display a Brookfield viscosity of about 15000 mPa.s after addition of 1,5% of NaCl.

A milder formulation with a total surfactant concentration of 9wt% containing 7% LES and 2% CAPB displays a viscosity lower than 5mPa.s after addition of 1% of NaCl. After addition of 1,5%NaCl, the viscosity is still lower than 100mPa.s

The viscosity effect of some of the polymers of table 1 was consequently tested in the following basic formulation:

**Formulation 1: Basic Cleansing formulation with 1 % Polymer of table 1, 7% LES, 2% CAPB und 1% NaCl:**

| | |
|---|---|
| A) | 50g polymer according to example 5 |
| B) | 35g of a 20% LES- solution |
| C) | 8g of a 25% CAPB-solution |
| | 2g water |
| D) | 4g of a 25% NaCl-solution |

Further components: q.s. perfume oil and preservative (Euxyl K100).

### Preparation:

Phases A and B were stirred together for about 2-3 hours at a temperature of 60°C until a clear solution was obtained. Phase C and 2g water were stirred into the mixture at 60°C and briefly after-homogenized: an increase in viscosity took place. Phase D was stirred into the mixture at 60°C and briefly after-homogenized: a dramatic increase in viscosity took place. The mixture was cooled down to about 30°C before adding the perfume oil (about 3 drops) and 0,1g of preservative. It was further stirred to cool it down to room temperature.

**Table 1:**

| **Water-soluble copolymer:** | **VP** | **VI** | **LUMA** | **MAS** | **MA** | **PETAE** | **Viscosity of formulation 1** |
|---|---|---|---|---|---|---|---|
| A (comparative example) | 60,4 | 36,0 | -- | 3,6 | -- | 0.30% | < 1000 mPas |
| B (comparative example without any polymer) | -- | -- | -- | -- | -- | -- | <5mPa.s |
| 1 | 56.8 | 36,0 | 3,6 | 3,6 | -- | 0.3 **) | ∼ 2000 mPas |
| 2 | 52,0 | 36,0 | 6,0 | 6,0 | -- | 0.3% | 2200 mPas |
| 3 | 38,0 | 50,0 | 6,0 | 6,0 | -- | 0.3% | 2500 mPas |
| 4 | 56.8 | 36,0 | 3,6 | 3,6 | -- | 0.15% | 9700 mPas |
| 5 | 56.8 | 36,0 | 3,6 | 3,6 | -- | -- | 16200 mPas |
| 6 | 56.8 | 36,0 | 3,6 | 3,6 | -- | -- | 16750 mPas |
| 7 | 26,8 | 36,0 | 3,6 | 3,6 | 30,0 | -- | 23400 mPas |
| 8 | 38,0 | 50,0 | 6,0 | 6,0 | | 0.05% | 14700 mPas |

## Claims

1. Use of a water-swellable copolymer consisting essentially of the following monomers in polymerized form
a) vinylpyrrolidone and/or methacrylamide (monomer A);
b) one or more monomers of the formula I
R¹-O-(CH₂-CHR-O)ₙ-R² (I)
wherein,
R¹ is a (meth)acrylic radical,
R is H or CH₃,
R² is a linear or branched C₈ to C₃₀ saturated hydrocarbon radical,
n is an integer of from 5 to 100
(monomer B);
c) vinylimidazole, quaternized vinylimidazole, N,N-dimethylaminopropyl-methacrylamide, quaternized N,N-dimethylaminoethyl (meth)acrylate, trimethylaminoethylmethacrylate chloride or a mixture of two or more thereof (monomer C);
d) (meth)acrylic acid and/or methyl(meth)acrylate (monomer D);
e) from 0 to 0.5% by weight of one or more cross-linkers (monomer E), based on the total weight of the monomers A to E,
as a rheology-modifier in a formulation comprising at least one surfactant and/or an oil.

2. Use according to claim 1 wherein the formulation is a cosmetic formulation.

3. Use according to claim 1 or 2 wherein the formulation is a shampoo or a body wash.

4. Use according to any one of claims 1 to 3 wherein the copolymer essentially consists of
a) from 40 to 75% by weight of monomer A;
b) from 0.5 to 20% by weight of monomer B;
c) from 10 to 60% by weight of monomer C;
d) from 0.5 to 20% by weight of monomer D;
e) from 0 to 0.25% by weight of monomer E,
based on the total weight of the monomers A to E.

5. Use according to any one of claims 1 to 4 wherein the copolymer essentially consists of
a) from 50 to 65% by weight of monomer A;
b) from 1 to 10% by weight of monomer B;
c) from 30 to 45% by weight of monomer C
d) from 1 to 10% by weight of monomer D;
e) from 0 to 0.2% by weight of monomer E,
based on the total weight of the monomers A to E.

6. Use according to any one of claims 1 to 5 wherein the amount of monomer E is from 0 to 0.15% by weight based on the total weight of the monomers A to E.

7. Use according to any one of claims 1 to 6 wherein monomer A is vinylpyrrolidone.

8. Use according to any one of claims 1 to 7 wherein monomer B is one or more monomers of the formula I
R¹-O-(CH₂-CHR-O)ₙ-R² (I)
wherein
R¹ is a methacrylic radical,
R is H,
R² is a linear or branched C₈-C₂₂ saturated hydrocarbon radical,
n is an integer from 10 to 30.

9. Use according to any one of claims 1 to 8 wherein monomer C is vinylimidazole and/or quaternized vinylimidazole.

10. Process for the preparation of water-swellable copolymers wherein the monomers are polymerized by means of precipitation polymerization in cyclohexane and/or ethyl acetate.

11. Cosmetic formulation comprising
α ) at least one water-soluble copolymer as defined in any one of claims 1 to 10,
β ) at least one surfactant,
γ ) optionally, one or more cosmetically acceptable active ingredient and/ or auxiliary different from α)

12. Cosmetic formulation according to claim 11 wherein the cosmetic formulation is a shampoo.

13. Cosmetic formulation according to claim 11 wherein the cosmetic formulation is a body wash.
